Europäisches Patentamt

European Patent Office

Office européen des brevets

(19)

(11) **EP 0 537 210 B1**

(12) **EUROPEAN PATENT SPECIFICATION**

(45) Date of publication and mention
of the grant of the patent:
**29.10.1997 Bulletin 1997/44**

(21) Application number: **91911767.1**

(22) Date of filing: **03.07.1991**

(51) Int Cl.$^6$: **G01N 33/18**

(86) International application number:
**PCT/AU91/00287**

(87) International publication number:
**WO 92/01223 (23.01.1992 Gazette 1992/03)**

(54) **CONTINUOUS RBCOD MEASUREMENT**

KONTINUIERLICHE RBCOD-MESSUNG

MESURE CONTINUE DE DCOBTR

(84) Designated Contracting States:
**AT BE CH DE DK ES FR GB GR IT LI LU NL SE**

(30) Priority: **04.07.1990 AU 974/90**

(43) Date of publication of application:
**21.04.1993 Bulletin 1993/16**

(73) Proprietor: **COMMONWEALTH SCIENTIFIC AND
INDUSTRIAL RESEARCH ORGANISATION
Campbell, ACT 2601 (AU)**

(72) Inventor: **BRIDGER, John, Stephen
Croydon, VIC 3136 (AU)**

(74) Representative: **Wharton, Peter Robert et al
Urquhart-Dykes & Lord
Inventions House
Valley Court, Canal Road
Bradford BD1 4SP (GB)**

(56) References cited:
**DE-A- 2 952 343          GB-A- 2 184 110
US-A- 4 564 453**

- **GWF DAS GAS-UND WASSERFACH, vol. 126,
  no.8, August 1985 MUNCHEN DE, pages 397-405,
  XP 000051699 U. SOLLFRANK, ET AL.
  'KONTINUIERLICHE MESSUNG DER
  RESPIRATION IN BELEBUNGSVERFAHREN'**
- **AUSTRALIAN WATER AND WASTEWATER
  ASSOCIATION 13TH FEDERAL CONVENTION, 6
  March 1989 - 10 March 1989 CANBERRA, AU,
  pages 294-298, K.C. LINDREA, ET AL. 'THE
  DETERMINATION OS THE READILY
  BIODEGRADABLE COD FRACTION OF
  WASTEWATER'**
- **PATENT ABSTRACTS OF JAPAN vol. 8 no. 215
  (P-305) [1652] ,2 October 1984 & JP-A-59 099353
  (KOGYO GIJUTSUIN ) 8 June 1984,**
- **PATENTS ABSTRACTS OF JAPAN, P-305, page
  47, JP,A, 59-99353 (KOGYO GIJUTSUIN) 8 June
  1984 (08.06.84).**
- **PATENTS ABSTRACTS OF JAPAN, P-156, page
  153, JP,A, 57-135357 (TOKYO SHIBAURA DENKI
  K.K.) 20 August 1982 (20.08.82).**
- **PATENTS ABSTRACTS OF JAPAN, P-58, page
  60, JP,A, 56-12546 (AJINOMOTO K.K.) 6
  February 1981 (06.02.81).**
- **PATENTS ABSTRACTS OF JAPAN, P-89, page
  164, JP,A, 56-108951 (NITSUSHIN DENKI K.K.) 28
  August 1981 (28.08.81).**
- **PATENTS ABSTRACTS OF JAPAN, P-511, page
  102, JP,A, 61-129567 (SHUKUJI ASAKURA) 17
  June 1986 (17.06.86).**
- **M.A. WINKLER, "Biological Treatment of
  Waste-Water", published 1981, Ellis Horwood
  Ltd (Chichester), see pages 160-161, Fig. 5.6.**

## Description

This invention relates to the measurement of readily biodegradable chemical oxygen demand (RBCOD) in an effluent or wastewater. In particular, the invention relates to a method and apparatus for real-time monitoring of RBCOD in a wastewater stream or feed.

The RBCOD of a waste stream is important as it may affect the operation of a process for treating the waste. For example, in biological sewage treatment systems capable of excess phosphorus removal, it has been shown that an appropriate portion of the incoming chemical oxygen demand (COD) needs to be readily biodegradable (Fuhs, G.W. and Chen, M. (1975). "Phosphorus Removal in Activated Sludge Process", Microbial. Ecology, 2, 119-139; Venter, S. L.V., Halliday, J. and Pitman, A.K. (1978). "Optimization of the Johannesburg Olifantzvlei Extended Aeration Plant for Phosphorus Removal", Prog Wat Tech, 10, 279-292). Where the RBCOD portion of the influent is found to be so low as not to achieve biological phosphorus removal, enrichment of the influent with fermentation products, such as volatile fatty acids (VFA) will be necessary. These products may come from solids settled from the sewage or from an external source such as digester sludge, industrial or agricultural waste.

The need for a continuous on-line method of monitoring the RBCOD in the feed to a sewage treatment plant for process control has been recognised. For example H.A. Nichols, C S Stevens and S Deacon in their paper "Full Scale Experimentation: Comparison of Different Control Strategies" published in the Papers of Technology Transfer Symposium "Advances in Biological Phosphorous Removal by the Activated Sludge Process" 27 October 1988 Water Research Commission of South Africa, state the following:-

"There is an urgent need ... to develop a good and reliable method of monitoring the readily biodegradable COD in the [sewage] feed, so that not only the performance of primary sedimentation tanks can be monitored, but also the performance of the activated sludge process itself."

RBCOD measurements are also useful to monitor the performance of treatment processes, whether or not they have been designed for excess phosphorus removal. Thus there is a need for measurement of the RBCOD of wastewater both up-stream and down-stream of a treatment plant. Furthermore the RBCOD of an effluent stream may assist in characterising that stream for design of a suitable treatment plant therefor.

Knowledge of RBCOD levels is also useful for the control of anaerobic digesters where an increase in RBCOD may indicate microbial imbalance within the digester.

## Background Art

Both biological and physical methods are known for measurement of RBCOD. Physical methods involving COD measurement of membrane filtered samples have given poor correlation with biological methods. The three main biological methods are 1) the short sludge age, step fed reactor, 2) the batch aerobic reactor and 3) the batch anaerobic reactor. These are described in some detail by Dold et. al. "Comparison of Measurement Methods for Readily Biodegradable COD Fraction in Municipal Wastewater", IWPC, Durban, South Africa (1985).

The short sludge age step fed reactor method has been reported as not giving consistent results and as being tedious, difficult to operate and unsuitable for the determination of in situ generated RBCOD. Furthermore, to obtain an RBCOD value representative of a 24 hour period, a large refrigerated composite sample would have to be collected each day. Long periods, for example 24 hours, are required for each measurement. Although measurement times can be reduced to about two hours with a batch aerobic reactor, the sampling and sample storage requirements for use of these reactors limit their applicability. The usefulness of anaerobic batch reactors is also limited by their sampling requirements. Furthermore RBCOD measurements based on limited sampling may not provide an accurate profile of the RBCOD of a waste stream because of wide variations over the diurnal cycle. Thus existing biological tests to determine RBCOD are not suitable for on-line or real-time monitoring of the influent to a treatment process or the effluent therefrom because they require a long time (2-24 hours) to obtain a result and the taking and storage of samples.

## Disclosure of the Invention

An object of this invention is to provide a method and apparatus allowing relatively quick measurements of the RBCOD of a wastewater stream or feed to be periodically taken.

By virtue of the invention it is possible to obtain almost "real-time" measurements of the RBCOD of a wastewater feed or stream such that the invention may be applied for monitoring, control or other purposes. For example, apparatus according to the invention could be left unattended for a period for relatively frequent data collection.

According to the invention there is provided a method for periodically determining the readily biodegradable chemical oxygen demand (RBCOD) in a wastewater stream or feed comprising:

i) continuously feeding a sample representative of the real-time wastewater stream or feed to a sample volume whereby the feed rate is controlled to ensure an hydraulic retention time (HRT) in the sample volume sufficient for substantially complete oxidation of the readily biodegradable compounds,

ii) periodically passing air for predetermined periods through the sample volume,

iii) determining the oxygen consumption in the sample volume by measuring a change in the dissolved oxygen content while not passing air through the sample volume, and

iv) calculating an RBCOD value from each oxygen consumption measurement.

In accordance with the invention, it is not a requirement that sludge be added to the sample as in prior art RBCOD measurements. Thus the method can be used for monitoring sewers at any point in a sewage network.

The invention also provides apparatus for monitoring a wastewater stream or feed comprising:

i) a bio-reactor suitable for maintaining a completely mixed sample volume and for continually receiving a sample representative of the real-time wastewater feed,

ii) air injection means which may be periodically operated to pass air for a predetermined period through a waste water sample when contained in the bio-reactor,

iii) means for measuring the dissolved oxygen content of a sample within the bio-reactor to determine the oxygen consumption of the sample from which the readily biodegradable chemical oxygen demand (RBCOD) is calculable.

The bio-reactor (that is, the sample volume of the method aspect of the invention) is completely mixed and as the hydraulic retention time (HRT) is chosen to ensure the RBCOD is substantially oxidised in the reactor, then the concentration of RBCOD within the reactor at any point in time will be close to zero. (There will be some oxygen demand by the bacteria just for them to survive - this is called endogenous oxygen uptake, which in the practice of the invention, may be assumed to be constant.) It follows then that the oxygen consumption at any point in time is due to the incoming feed (plus the assumed constant endogenous demand).

An RBCOD value for each oxygen consumption measurement is calculated by multiplying the oxygen consumption measurement by a constant. Thus, soon after the start of an air-off period a first dissolved oxygen concentration measurement ($DO_1$) is taken and after a fixed period of time $t_p$ a second dissolved oxygen concentration measurement ($DO_2$) is taken from which the oxygen uptake consumption in the reactor in time $t_p$ is given by:

$$(DO_1 - DO_2) \times V = \Delta DO \times V \text{ mg}$$

where V (litres) is the sample volume.

The RBCOD may be calculated from the change in dissolved oxygen concentration over the set measuring period by multiplying the value obtained by an apparatus constant which may be determined by calculation from the parameters of the system which are held fixed or by calibration using acetate solutions of known concentration as is shown below. Thus

$$RBCOD = \Delta DO \times constant.$$

The value for the constant includes a conversion factor of 3 for converting oxygen consumption to RBCOD (as suggested by Dold et al, supra) and factors relating the oxygen consumption in the bio-reactor over time $t_p$ to oxygen consumption in the quantity of the feed that enters the reactor. These factors include the feed rate into the bio-reactor, a constant measurement period $t_p$ and the bio-reactor volume.

Brief Description of Drawings

The invention will now be described, by way of example only, with reference to the accompanying drawings in which:
Figure 1 is a diagrammatic sketch of apparatus according to the invention.
Figures 2 and 3 illustrate $O_2$ consumption of a batch reactor due to Acetate addition for comparison with and calibration of a reactor according to the invention.
Figures 4 and 5 illustrate $O_2$ consumption in a reactor according to the invention due to Acetate addition.

Figures 6 and 7 illustrate on-line operation of the method and apparatus of the invention.

Best Mode for Carrying Out the Invention

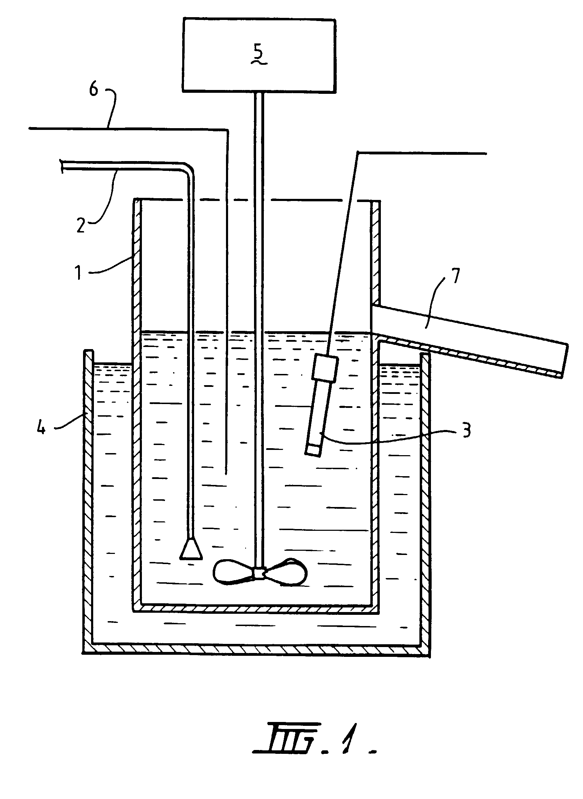

Figure 1 illustrates example apparatus according to the invention. The apparatus comprises a 1.4 litre bioreactor 1 with an air injection means 2 and dissolved oxygen measuring probe 3. Associated with the bioreactor are temperature control means, in this case a water bath 4 maintained a 20°C, and a stirring means, for example a magnetic stirrer 5. An influent feed line is shown at 6 and overflow at 7. A temperature control means may not be required, for example in applications of the invention where the ambient temperature does not vary widely. Also, the bio-reactor may conveniently include means to clean its internal surface, such as for example, a scraping means which is operable at selected intervals of time.

Apparatus according to the invention may be supplied as a self contained portable unit, which unit may also include a data processing means for providing electrical output signals representative of RBCOD determinations. Optionally, such a portable unit may also include a pump connected to the bio-reactor input for supplying a constant feed thereto.

Experimental Set Up

Two bio-reactors, each of 1.4 litres volume, were set up housed in a water bath at 20°C and continuously fed by a peristaltic pump. The reactors were stirred at about 60 rpm. Since no attempt was made to retain solids, they acted as completely mixed reactors with the sludge age equal to the hydraulic retention time (HRT) which was around six hours. Air was applied in a 15 minute on, 15 minute off cycle at a rate chosen to prevent oxygen saturation occurring. (Any suitable on and off period for the air supply may be chosen, for example an on and off cycle of between 1 and 30 minutes may be chosen.) Measurement of dissolved oxygen (DO) was made by polarographic electrode connected to a flat bed chart recorder. This method and apparatus by which relatively quick periodical measurements of RBCOD may be taken is referred to herein as a "continuous" method or "continuous" reactor.

The DO electrode's response rate is preferably very much greater and its oxygen consumption rate very much smaller than the maximum and minimum oxygen uptake rates to be measured. The electrodes used were Titron (Registered Trade Mark) 500MB with a response rate of around 2000mg/1/hr and a consumption rate of <0.1mg/1/hr. Maximum and minimum oxygen uptake rates in the reactors were in the order of 20 to 2 mg/1/hr.

So as to maintain a low endogenous oxygen uptake rate (OUR) in the reactor, cleaning of bio-film from all surfaces within the reactor was carried out once a day. The DO electrodes were likewise cleaned and calibrated to maintain their accuracy. Alternatively or additionally, the bio-reactor may include a cleaning means as has been described above.

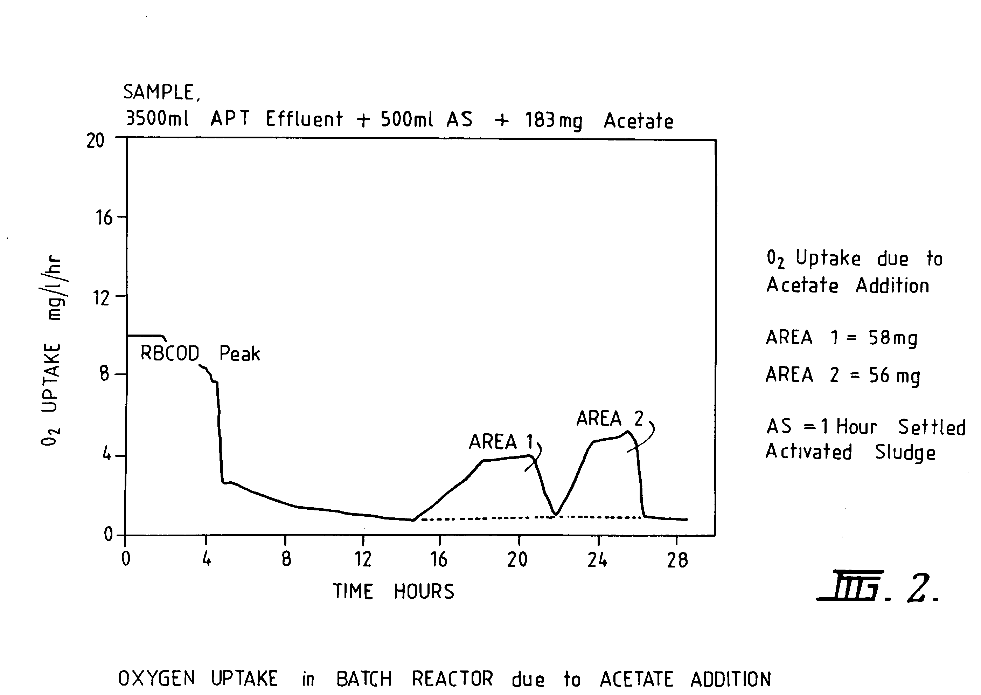

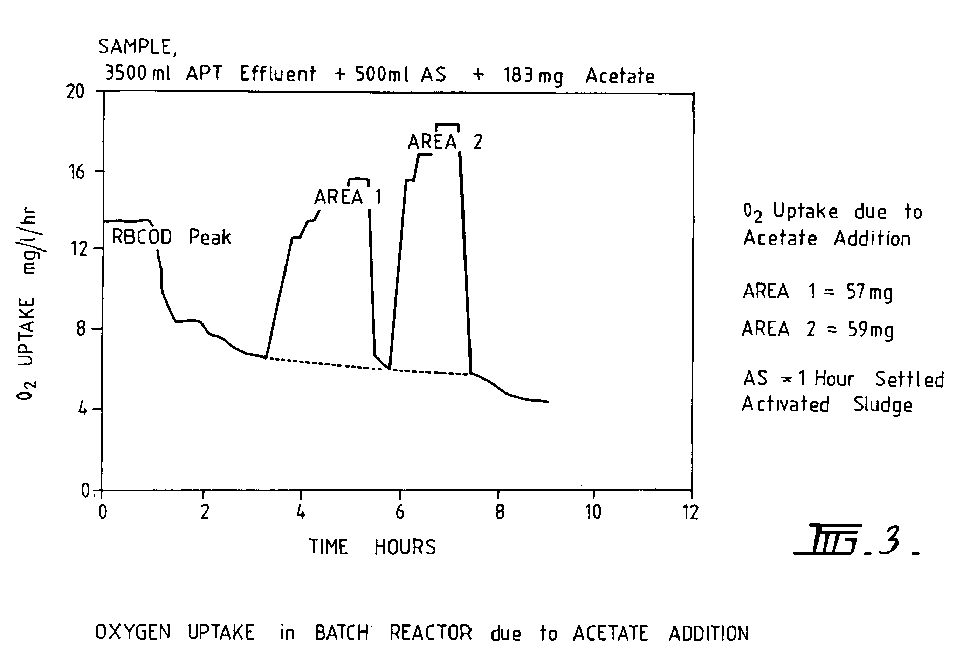

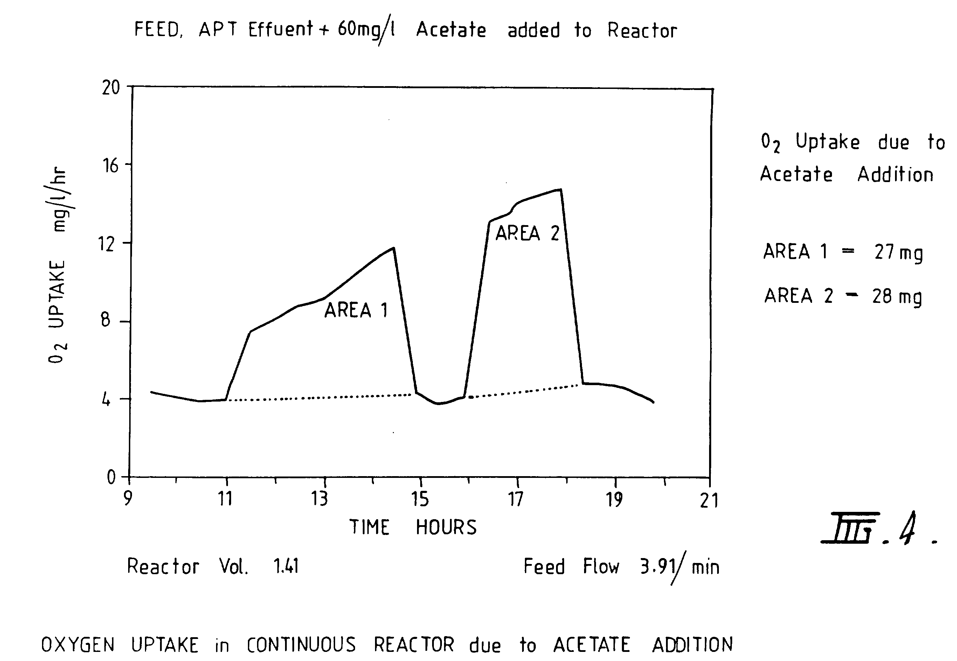

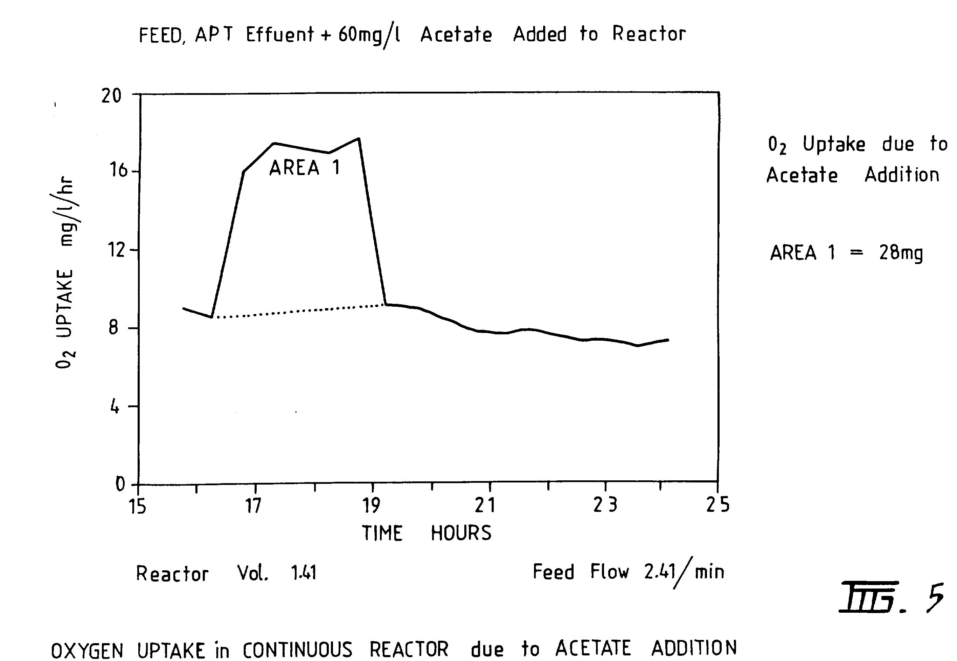

Calibration of the reactors. To calibrate the oxygen uptake response of the continuous reactors, a comparison was made with an established batch method (ref. Lindrea et al - see below) by the addition of sodium acetate, expressed as acetic acid, to both the continuous reactors and a batch reactor. Plots of oxygen consumption rates following additions of acetate are shown for the batch method in Figures 2 and 3, and for the continuous method in Figures 4 and 5.

Sequential additions of acetate to both batch and continuous reactors result in similar shaped peaks in so much as the consumption rate increases with time. This may indicate that the population of bacteria is increasing to meet the available substrate or the bacterial population is being "switched on" due to the stimuli of the substrate. The area under the curve, or oxygen consumption, is similar for sequential additions to both types of reactor. A surprising feature was the low rate of degradation of the acetate substrate in the batch reactor, considering that it had a high biomass content compared to the continuous reactor.

The measured oxygen consumption for each peak is shown in Table 1 as a percentage of the theoretical chemical oxygen demand of the acetate added. No allowance has been made in the calculation for the acetate lost due to wash out from the continuous reactor, estimated to be between 10 and 15% of the acetate added.

TABLE 1

| % of Chemical Oxygen Demand of Acetate | | | | | |
|---|---|---|---|---|---|
| | Batch | | | Continuous | |
| | Area 1 | Area 2 | | Area 1 | Area 2 |
| Fig 2 | 29% | 29% | Fig 4 | 29% | 31% |
| Fig 3 | 29% | 31% | Fig 5 | 30% | |

The above figures are in excellent agreement with each other, and when converted to RBCOD using the factor of 3 recommended by Dold et. al. (supra) give a result similar to that reported by Lindrea et. al. (1988) "The Determination of the Readily Biodegradable COD Fraction of Wastewater", Australian Water and Wastewater Association, 13th Federal Convention, Canberra, March 6-10, pp.294-298.

The possibility existed that oxygen adsorption from the atmosphere may be occurring through the open top continuous reactors during the air off period. This would result in lower oxygen uptake readings, although the acetate addition results did not suggest this. Tight fitting closed cell foam disks were pressed into the reactors down to liquid surface level. No measurable difference was found in the oxygen consumption rate and as the discs only increase the surface area to volume ratio for biofilm growth, they may be dispensed with.

It was felt that these results were sufficiently encouraging for the apparatus to be used for the measurement of RBCOD in the influent and effluent streams of an activated primary tank (APT) of a $5.3m^3$/day pilot sewage treatment plant operated by CSIRO at its Lower Plenty Research Station (Bayly et al "The Effect of Primary Fermentation on Biological Nutrient Removal" Australian Water and Wastewater Association, 13th Federal Convention, Canberra, March 6-10, 1989, pp. 162-166). Excess phosphorous removal was associated with the pretreatment of the pilot plant feed by the APT, the purpose of which is to increase the concentration of influent RBCOD.

Continuous In Situ Measurement of RBCOD

One reactor was fed with raw screened sewage continuously pumped from the feed stream to the APT, the other with APT effluent which is used to feed the Bio-P removal pilot plant.

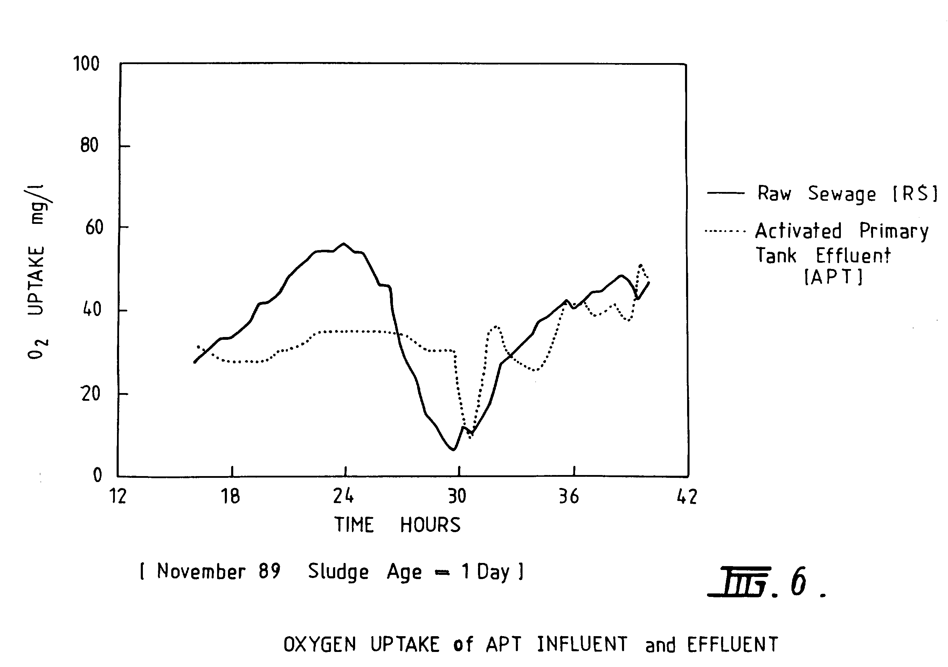

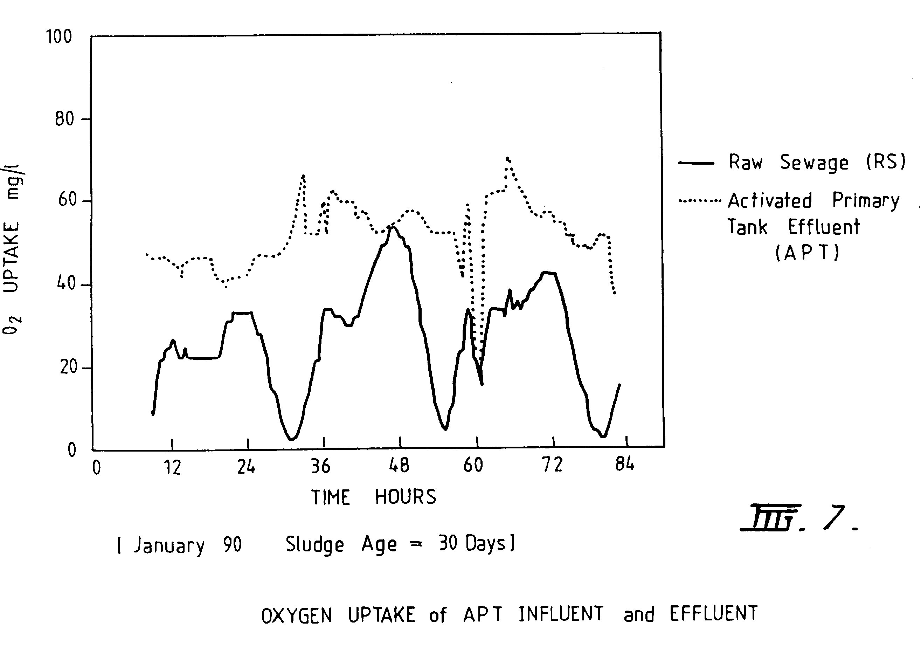

The data shown in Figure 6 is typical of that collected during November 1989 when the APT was operating as a clarifier, the settled solids being drawn off once a day to give a sludge age of approximately one day. Under these conditions it appears that very little if any RBCOD was being generated, the average oxygen consumption values are, $37mgO_2$/l of influent and $33mgO_2$/l of effluent. In contrast Figure 7 is typical of data collected during January 1990 when settled solids were being built up in the APT to obtain a sludge age of approaching 30 days. The average influent oxygen consumption for Figure 7 is $26mgO_2$/l and effluent $51mgO_2$/l.

The diurnal pattern of raw sewage oxygen consumption can be clearly seen in Figure 7. Typically a very low value occurs at about 6 to 7am followed by an initial peak at about mid-day, a plateau or trough, then a second usually higher peak around 10pm. The minimum diurnal value is in the region of 4 to $5mgO_2$/l and the maximum value between 40 and $60mgO_2$/l.

The effluent pattern is damped and moved in time by the APT hydraulic retention time HRT, which is variable with the diurnal feed rate. Also the feed rate to the APT dictates the degree of dilution of soluble substrates being produced from the accumulated settled solids. The dip in the curves at around 8am is caused by flow cessation for routine reactor cleaning.

The continuous fed reactor returns similar oxygen uptake results to the batch method when calibrated by acetate addition. The method of the invention provides a convenient investigative adjunct to the well established batch method and provides a clearer picture of diurnal and day to day RBCOD variations. This information could be used to predict the performance of an operating process with or without an APT or be used for design of new plants.

**Claims**

**1.** A method for periodically determining the readily biodegradable chemical oxygen demand (RBCOD) in a wastewater stream or feed comprising:-

i) continuously feeding a sample representative of the real-time wastewater stream or feed to a sample volume whereby the feed rate is controlled to ensure an hydraulic retention time (HRT) in the sample volume sufficient for substantially complete oxidation of the readily biodegradable compounds,

ii) periodically passing air for predetermined periods through the sample volume,

iii) determining the oxygen consumption in the sample volume by measuring the change in dissolved oxygen content ($\Delta DO$) over a fixed period of time ($t_p$) while not passing air through the sample volume;

iv) calculating an RBCOD value from the change in dissolved oxygen concentration ($\Delta DO$) over the measuring period $t_p$ by multiplying the value obtained by an apparatus constant determined either by calculation from the parameters of the system which are held fixed or by calibration using acetate solutions of known concentration.

**2.** A method as claimed in claim 1 wherein the sample volume is maintained at a substantially constant temperature.

**3.** A method as claimed in claim 1 wherein the sample volume is continuously stirred.

4. A method is claimed in claim 1 wherein the predetermined period for each periodical passage of air through the sample volume together with the air-off period is within the range of between 1 to 30 minutes.

5. A method as claimed in claim 4 wherein the predetermined period plus the air-off period is between 10 to 15 minutes.

6. Apparatus for measuring the readily biodegradable chemical oxygen demand (RBCOD) in a wastewater stream or feed comprising:

   i) a bio-reactor suitable for maintaining a completely mixed sample volume and for continually receiving a sample representative of the real-time wastewater feed,

   ii) means for controlling the feed rate of a sample of the wastewater feed to the bio-reactor to ensure an hydraulic retention time (HRT) in the bio-reactor sufficient for substantially complete oxidation of readily biodegradable compounds,

   iii) air injection means in the bio-reactor which may be periodically operated to pass air for a predetermined period through a wastewater sample when contained in the bio-reactor,

   iv) means for measuring the dissolved oxygen content of a sample within the bio-reactor to determine the oxygen consumption of the sample from which the readily biodegradable chemical oxygen demand (RBCOD) is calculable.

7. Apparatus as claimed in claim 6 including means to maintain a sample volume at a substantially constant temperature.

8. Apparatus as claimed in claim 6 or claim 7 including means for stirring the bio-reactor contents.

9. Apparatus as claimed in any one of claims 6 to 8 wherein the bio-reactor includes an inlet and an overflow outlet, the inlet being connectable to receive a wastewater feed.

10. Apparatus as claimed in claim 6 wherein the bio-reactor includes means to clean the internal surface of the reactor.

11. Apparatus as claimed in claim 10 wherein the inlet is connected to receive a continual wastewater feed.

12. A self contained portable unit for measuring RBCOD comprising apparatus as claimed in any one of claims 6 to 10 housed with the unit together with a data processing means for providing electrical output signals representative of RBCOD determinations.

13. A self contained portable unit as claimed in claim 12 also including a pump, for supplying a feed to the bio-reactor, housed within the unit.

**Patentansprüche**

1. Verfahren zur periodischen Bestimmung des biologisch leicht abbaubaren chemischen Sauerstoffbedarfs (RBCOD, readily biodegradable chemical oxygen demand) in einem Abwasserstrom bzw. -einsatzstrom, bei dem man:

   i) einem Probenvolumen eine Durchschnittsprobe des Echtzeit-Abwasserstroms bzw. -einsatzstroms kontinuierlich zuführt, wobei die Zulaufrate so gesteuert wird, daß eine zur weitgehend vollständigen Oxidation der biologisch leicht abbaubaren Verbindungen ausreichenden hydraulischen Verweilzeit (HVZ) im Probenvolumen gewährleistet ist,
   ii) periodisch über vorbestimmte Zeiträume Luft durch das Probenvolumen hindurchleitet,
   iii) den Sauerstoffverbrauch im Probenvolumen bestimmt, indem man die Veränderung des Gehalts an gelöstem Sauerstoff ($\Delta DO$) über einen festen Zeitraum $t_p$ mißt, wenn keine Luft durch das Probenvolumen hindurchgeleitet wird;
   iv) aus der Veränderung der Konzentration an gelöstem Sauerstoff ($\Delta DO$) über den Meßzeitraum $t_p$ einen RBCOD-Wert berechnet, indem man den erhaltenen Wert mit einer entweder durch Berechnung aus den festgelegten Systemparametern oder durch Kalibrierung mit Acetatlösungen bekannter Konzentration bestimmten Apparatekonstante multipliziert.

2. Verfahren nach Anspruch 1, bei dem man die Temperatur des Probenvolumens weitgehend konstant hält.

3. Verfahren nach Anspruch 1, bei dem man das Probenvolumen kontinuierlich rührt.

4. Verfahren nach Anspruch 1, bei dem die Summe aus dem vorbestimmten Zeitraum für jedes periodische Durchleiten von Luft durch das Probenvolumen und dem Zeitraum, über den keine Luft durchgeleitet wird, 1 bis 30 Minuten beträgt.

5. Verfahren nach Anspruch 4, bei dem die Summe aus vorbestimmtem Zeitraum und dem Zeitraum, über den keine Luft durchgeleitet wird, 10 bis 15 Minuten beträgt.

6. Vorrichtung zur Messung des biologisch leicht abbaubaren chemischen Sauerstoffbedarfs (RBCOD) in einem Abwasserstrom bzw. -einsatzstrom mit:

   i) einem Bioreaktor, der das Probenvolumen vollständig durchmischt halten und eine Durchschnittsprobe des Echtzeit-Abwassereinsatzstroms kontinuierlich aufnehmen kann,
   ii) einer Einrichtung zur Steuerung der Zulaufrate einer Probe des Abwassereinsatzstroms zum Bioreaktor zur Gewährleistung einer zur weitgehend vollständigen Oxidation der biologisch leicht abbaubaren Verbindungen ausreichenden hydraulischen Verweilzeit (HVZ),
   iii) einer Einrichtung zum Einleiten von Luft in den Bioreaktor, mit der periodisch über einen vorbestimmten Zeitraum Luft durch eine sich im Bioreaktor befindende Abwasserprobe hindurchgeleitet werden kann,
   iv) einer Einrichtung zur Messung des Gehalts einer sich im Bioreaktor befindenden Probe an gelöstem Sauerstoff zwecks Bestimmung des Sauerstoffverbrauchs der Probe, aus welchem sich der biologisch leicht abbaubare chemische Sauerstoffbedarf (RBCOD) berechnen läßt.

7. Vorrichtung nach Anspruch 6 mit Einrichtungen zum Halten des Probenvolumens bei einer weitgehend konstanten Temperatur.

8. Vorrichtung nach Anspruch 6 oder 7 mit einer Einrichtung zum Rühren des Inhalts des Bioreaktors.

9. Vorrichtung nach einem der Ansprüche 6 bis 8, wobei der Bioreaktor einen Einlaß und einen Überlauf enthält und der Einlaß an einen Abwassereinsatzstrom angeschlossen werden kann.

10. Vorrichtung nach Anspruch 6, wobei der Bioreaktor Einrichtungen zur Reinigung der Reaktorinnenwand enthält.

11. Vorrichtung nach Anspruch 10, wobei der Einlaß an einen kontinuierlichen Abwassereinsatzstrom angeschlossen ist.

12. Autonomes tragbares Gerät zur Bestimmung des RBCOD, in dem eine Vorrichtung nach einem der Ansprüche 6 bis 10 sowie eine Datenverarbeitungseinrichtung zur Lieferung von den RBCOD-Bestimmungen entsprechenden elektrischen Ausgangssignalen untergebracht sind.

13. Autonomes tragbares Gerät nach Anspruch 12, in dem außerdem noch eine Pumpe zur Zufuhr eines Einsatzstoffs zum Bioreaktor untergebracht ist.

**Revendications**

1. Méthode pour déterminer périodiquement la demande chimique en oxygène biodégradable très rapidement (DCOBTR) dans un courant ou une alimentation d'eaux usées comprenant les étapes consistant à:

   i) alimenter en continu un volume d'échantillon par un échantillon représentatif du courant ou de l'alimentation d'eaux usées en temps réel, le débit d'alimentation étant contrôlé pour assurer un temps de rétention hydraulique (TRH) dans le volume d'échantillon suffisant pour une oxydation sensiblement complète des composés biodégradables très rapidement,
   ii) faire passer périodiquement de l'air pendant des périodes prédéterminées à travers le volume d'échantillon,

iii) déterminer la consommation en oxygène dans le volume d'échantillon en mesurant la variation de la teneur en oxygène dissous ($\Delta DO$) sur une période de temps fixe ($t_p$) tout en ne faisant pas passer d'air à travers le volume d'échantillon;

iv) calculer une valeur de DCOBTR à partir de la variation de la teneur eu oxygène dissous ($\Delta DO$) sur la période de mesure $t_p$ en multipliant la valeur obtenue par une constante d'appareil déterminée soit par calcul à partir des paramètres du système qui sont maintenus fixes soit par étalonnage en utilisant des solutions d'acétate de concentrations connues.

2. Méthode selon la revendication 1, dans laquelle le volume d'échantillon est maintenu à une température essentiellement constante.

3. Méthode selon la revendication 1, dans laquelle le volume d'échantillon est agité en continu.

4. Méthode selon la revendication 1, dans laquelle la période prédéterminée pour chaque passage périodique d'air à travers le volume d'échantillon conjointement avec la période sans air est dans la gamme comprise entre 1 et 30 minutes.

5. Méthode selon la revendication 4, dans laquelle la période prédéterminée plus la période sans air est comprise entre 10 et 15 minutes.

6. Appareil de mesure de la demande chimique en oxygène biodégradable très rapidement (DCOBTR) dans un courant ou une alimentation d'eaux usées comprenant:

i) un bioréacteur approprié pour maintenir un volume d'échantillon complètement mélangé et pour recevoir en continu un échantillon représentatif de l'alimentation d'eaux usées en temps réel,

ii) un moyen pour contrôler le débit d'alimentation d'un échantillon de l'alimentation d'eaux usées au bioréacteur pour assurer un temps de rétention hydraulique (TRH) dans le bioréacteur suffisant pour une oxydation sensiblement complète de composés biodégradables très rapidement,

iii) un moyen d'injection d'air dans le bioréacteur qui peut être périodiquement actionné pour faire passer de l'air pendant une période prédéterminée à travers un échantillon d'eaux usées lorsqu'il est contenu dans le bioréacteur,

iv) un moyen pour mesurer la teneur en oxygène dissous d'un échantillon à l'intérieur du bioréacteur en vue de déterminer la consommation en oxygène de l'échantillon à partir de laquelle la demande chimique en oxygène biodégradable très rapidement (DCOBTR) est calculable.

7. Appareil selon la revendication 6, comprenant un moyen pour maintenir un volume d'échantillon à une température sensiblement constante.

8. Appareil selon la revendication 6 ou la revendication 7, comprenant un moyen pour agiter le contenu du bioréacteur.

9. Appareil selon l'une quelconque des revendications 6 à 8, dans lequel le bioréacteur comprend une entrée et une sortie de trop-plein, l'entrée pouvant être connectée pour recevoir une alimentation d'eaux usées.

10. Appareil selon la revendication 6, dans lequel le bioréacteur comprend un moyen pour nettoyer la surface interne du réacteur.

11. Appareil selon la revendication 10, dans lequel l'entrée est connectée pour recevoir une alimentation d'eaux usées continue.

12. Unité portative autonome pour mesurer la DCOBTR comprenant un appareil selon l'une quelconque des revendications 6 à 10 logé à l'intérieur de l'unité conjointement avec un moyen de traitement de données pour fournir des signaux de sortie électriques représentatifs des déterminations de la DCOBTR.

13. Unité portative autonome selon la revendication 12, comprenant également une pompe, pour fournir une alimentation au bioréacteur, logée à l'intérieur de l'unité.

$$\overline{\overline{III}}.\,1\,.$$

SAMPLE,
3500ml APT Effluent + 500ml AS + 183mg Acetate

OXYGEN UPTAKE in BATCH REACTOR due to ACETATE ADDITION

$O_2$ Uptake due to Acetate Addition

AREA 1 = 58mg

AREA 2 = 56 mg

AS = 1 Hour Settled Activated Sludge

FIG. 2.

EP 0 537 210 B1

OXYGEN UPTAKE in BATCH REACTOR due to ACETATE ADDITION

Fig. 3.

FEED, APT Effuent + 60mg/l Acetate added to Reactor

$O_2$ Uptake due to Acetate Addition

AREA 1 = 27 mg

AREA 2 = 28 mg

FIG. 4.

OXYGEN UPTAKE in CONTINUOUS REACTOR due to ACETATE ADDITION

EP 0 537 210 B1

FEED, APT Effuent + 60mg/l Acetate Added to Reactor

OXYGEN UPTAKE in CONTINUOUS REACTOR due to ACETATE ADDITION

$O_2$ Uptake due to Acetate Addition

AREA 1 = 28mg

Reactor Vol. 1.4l          Feed Flow 2.4 l/min

FIG. 5

EP 0 537 210 B1

[ November 89   Sludge   Age = 1 Day ]

FIG. 6.

OXYGEN UPTAKE of APT INFLUENT and EFFLUENT

[ January 90    Sludge Age = 30 Days]

FIG. 7.

OXYGEN UPTAKE of APT INFLUENT and EFFLUENT

EP 0 537 210 B1